# EUROPEAN PATENT APPLICATION

(11) **EP 1 614 417 A1**
(43) Date of publication of application: **11.01.2006**
(21) Application number: 04727412.1
(22) Date of filing: 14.04.2004
(51) Int. Cl.: A61K 31/195, A61K 47/12, A61K 47/26, A61K 47/38, A61K 47/44, A61K 9/00

(54) **SOLID PREPARATION FOR ORAL USE**

(30) Priority: 16.04.2003 JP 2003111482
(71) Applicant: KYORIN PHARMACEUTICAL CO., LTD., Chiyoda-ku, Tokyo 101-0062 (JP)
(72) Inventor: HOSHINO, Ryouichi, Shimotsuga-gun, Tochigi 329-0100 (JP); NAKASHIMA, Katashi, Tatebayashi-shi, Gunma 374-0016 (JP); FUKUDA, Mamoru, Shimotsuga-gun, Tochigi 329-0114 (JP)
(74) Representative: Merkle, Gebhard
(86) International application number: PCT/JP2004/005317
(87) International publication number: WO 2004/091600

(57) **Abstract**

Upon performing the clinical study of (S)-2-[3-[N-[4-(4-fluorophenoxy)benzyl]carbamoyl]-4-methoxybenz yl]butanoic acid (hereinafter abbreviated as KRP-101), of which improvement in the lipidmetabolism is expected in a microdose, no oral solid dosage form that allows KRP-101 to be administered quantitatively has been embodied.

After mixing KRP-101 with additives (excipient, disintegrator and lubricant), the mixture is granulated, pressed into tablets and coated with coating agent, thereby film-coated tablets uniformly containing a small amount of KRP-101 are obtained, making it possible to administer a microdose of KRP-101 quantitatively on clinical study.

## Description

### Technical field

The present invention relates to an oral solid dosage form (tablet) containing (S)-2-[3-[N-[4-(4-fluorophenoxy)benzyl]carbamoyl]-4-methoxybenz yl]butanoic acid (hereinafter abbreviated as KRP-101), of which improvement in the lipidmetabolism is expected, in a small amount and yet uniformly, and process for preparing it.

### Background technology

[Patent document 1] Jpn. Kokai Tokkyo Koho JP 2001-55367 KRP-101 is publicly known as a derivative of substituted phenylpropanoic acid ([Patent document 1]), and is under development as a lipidmetabolism-improving agent that exhibits an agonistic activity on human peroxisome proliferant-activated receptor and has lowering effects for both cholesterol and triglyceride in a microdose.

Upon performing the clinical study of KRP-101, no oral solid dosage form that allows KRP-101, of which improvement in the lipidmetabolism is expected in a microdose, to be administered quantitatively, has been embodied.

### Disclosure of the invention

After mixing KRP-101 with additives (excipient, disintegrator and lubricant), the mixture is granulated and, by compressing the granules into tablets and coating on to the prime tablets obtained with coating agent ,film-coated tablets uniformly containing a small amount of KRP-101 are obtained, making it possible to provide an oral solid dosage form that allows a microdose of KRP-101 to be administered quantitatively on clinical study.

Namely, the invention relates to:
1) An oral solid dosage form having KRP-101 as an active ingredient and comprising KRP-101 and additives;
2) The oral solid pharmaceutical of 1), wherein the additives comprise excipient, disintegrator and lubricant, or these and coating agent;
3) The oral solid dosage form of 1) or 2), wherein the excipient comprises lactose and/or microcrystalline cellulose, the disintegrator comprises low substituted hydroxypropylcellulose, the lubricant comprises magnesium stearate, and the coating agent comprises hydroxypropylmethylcellulose and/or carnauba wax;
4) The oral solid dosage form of any of 1) through 3), wherein, to a mixed powder obtained by repeating a plurality of steps of mixing and dilution of KRP-101 with excipient, the excipient, disintegrator and lubricant are added and the mixed powder with less than 1% of KRP-101 is granulated.

The process for preparing the oral solid dosage form of the invention is as follows: After the excipients (for example, saccharides such as lactose and glucose, sugar alcohols such as D-sorbitol and mannitol, celluloses such as microcrystalline cellulose, starches such as corn starch, preferably lactose and microcrystalline cellulose), the disintegrator (for example, celluloses such as low substituted hydroxypropylcellulose, carmellose calcium, croscarmellose sodium, crospovidone, partly pregelatinized starch, etc., preferably low substituted hydroxypropylcellulose), and the lubricant (for example, magnesium stearate, calcium stearate, talc, hydrogenated oil, etc., preferably magnesium stearate) are mixed, the mixture is granulated with dry granulation process. The obtained granules are compressed into tablets, and the coating agents (for example, celluloses such as hydroxypropylcellulose, hydroxypropylmethylcellulose, ethylcellulose and methylcellulose, hydroxypropylmethylcellulose phthalate, methacrylic copolymer, titanium dioxide, iron sesquioxide, carnauba wax, etc., preferably hydroxypropylmethylcellulose and carnauba wax) are coated onto the plain tablets.

For quantitatively administering a microdose of KRP-101, it is required to make the content of KRP-101 in the oral solid dosage form more uniform. For this, following preparative process is preferable. Aggregates contained in a small amount in the powder of KRP-101 are extruded forcibly to pass through a sieve with 177 - µm mesh. To 1 part by weight of KRP-101 passed through the sieve, 4 parts by weight of excipient are added and mixed (= mixed powder A). To 1 part by weight of mixed powder A, 1part by weight of excipient is added and mixed (= mixed powder B) . After weighing the mixed powder B thus obtained, excipient, disintegrator and lubricant so as to become desirable content of KRP-101, these are mixed (= mixed powder C). The mixed powder C is granulated with dry granulation process, and the obtained granules are compressed into tablets, to make plain tablets. Coating is given thereto to prepare the aimed film-coated tablets.

Since the film-coated tablets obtained in this way uniformly contain small amount of around 0.025 to 1 mg of KRP-101 per tablet, they allow quantitative oral administration.

### Best mode for carrying out the invention

In following, the invention will be illustrated in detail based on the examples, but the invention is not confined to these examples.

### <Example 1>

KRP-101 passed through a sieve with 177-µm mesh and lactose (75 µm sieve-passed article) were weighed in amounts of 11 and 44 g, respectively, and mixed for 20 minutes in Mechanomill (Okada Seiko Co. , Ltd. , Model MM-10N) . To this, 55 g of lactose (75 µm sieve-passed article) were added and mixed further for 20 minutes. The mixed powder thus obtained, lactose (75 µm sieve- passed article) , microcrystalline cellulose and low substituted hydroxypropylcellulose were weighed in amounts of 80, 725.6, 265.6 and 120 g, respectively, and mixed for 10 minutes in High-Speed Mixer (Fukae Kogyo Co., Ltd., Model FSGS-5) . To this, 8.8 g of magnesium stearate were added and mixed further for 5 minutes. The mixed powder obtained was molded into flakes in shape under a pressure of 100 kgf/cm² with Roller Compactor (Freund Industrial Co., Ltd., Model TF-MINI) and these flakes were pulverized with Roll Granulator (Nippon Granulator Co., Ltd., Model GRN-T-54-S) to obtain granules. These granules were submitted to compression molding with tabletting machine (Hata Iron Works Co., Ltd., Model HT-AP18SS-II) to obtain prime tablets having weight of one tablet of 150 mg and shape of diameter and radius of curvature of 7 and 9 mm, respectively. After an aqueous solution of hydroxypropylmethylcellulose was coated so as to coat 5 mg of hydroxypropylmethylcellulose 2910 per tablet obtained, 0.001 mg of carnauba wax was added and mixed to obtain film-coated tablets containing 1 mg of KRP-101.

### <Example 2>

KRP-101 passed through a sieve with 177-µm mesh and lactose (75 µm sieve-passed article) were weighed in amounts of 12 and 48 g, respectively, and mixed for 20 minutes in Mechanomill (Okada Seiko Co., Ltd., Model MM-10N) . To this, 60 g of lactose (75 µm sieve-passed article) were added and mixed further for 20 minutes. The mixed powder thus obtained, lactose(75 µm sieve-passed article), microcrystalline cellulose and low substituted hydroxypropylcellulose were weighed in amounts of 50, 4970, 1675 and 750 g, respectively, and mixed for 10 minutes in High-Speed Mixer (Fukae Kogyo Co., Ltd. , Model FS-20). To the mixed powder obtained, 110 g of magnesium stearate were added and mixed further for 5 minutes with Powmixer (Tsukasa Kogyo Co., Ltd., Model PM-V-80-S). The mixed powder obtained was molded into flakes in shape under a pressure of 100 kgf/cm² with Roller Compactor (Freund Industrial Co., Ltd., Model TF-MINI) and these flakes were pulverized with Roll Granulator (Nippon Granulator Co., Ltd., Model GRN-T-54-S) to obtain granules. These granules were submitted to compression molding with tabletting machine (Hata Iron Works Co., Ltd., Model HT-AP18SS-II) to obtain prime tablets having weight of one tablet of 150 mg and shape of diameter and radius of curvature of 7 and 9 mm, respectively. After an aqueous solution of hydroxypropylmethylcellulose was coated so as to coat 5 mg of hydroxypropylmethylcellulose 2910 per plain tablet, 0.001 mg of carnauba wax was added and mixed to obtain film-coated tablets containing 0.1 mg of KRP-101.

### <Example 3>

KRP-101 passed through a sieve with 177-µm mesh and lactose (75 µm sieve-passed article) were weighed in amounts of 11 and 44 g, respectively, and mixed for 20 minutes in Mechanomill (Okada Seiko Co. , Ltd., Model MM-10N) . To this, 55 g of lactose (75 µm sieve-passed article) were added and mixed further for 20 minutes. The mixed powder thus obtained, lactose (75 µm sieve-passed article), microcrystalline cellulose and low substituted hydroxypropylcellulose were weighed in amounts of 2,801.2, 268 and 120 g, respectively, and mixed for 10 minutes in High-Speed Mixer (Fukae Kogyo Co. , Ltd. , Model FSGS-5). To this, 8.8 g of magnesium stearate were added and mixed further for 5 minutes. The mixed powder obtained was molded into flakes in shape under a pressure of 100 kgf/cm² with Roller Compactor (Freund Industrial., Ltd., Model TF-MINI) and these flakes were pulverized with Roll Granulator (Nihon Granulator Co., Ltd., Model GRN-T-54-S) to obtain granules. These granules were submitted to compression molding with tabletting machine (Hata Iron Works Co., Ltd., Model HT-AP18SS-II) to obtain prime plain tablets having weight of one tablet of 150 mg and shape of diameter and radius of curvature of 7 and 9 mm, respectively. After an aqueous solution of hydroxypropylmethylcellulose was coated so as to coat 5 mg of hydroxypropylmethylcellulose 2910 per plain tablet obtained, 0.001 mg of carnauba wax was added and mixed to obtain film-coated tablets containing 0.025 mg of KRP-101.

### <Experimental example 1>

The content of KRP-101 in the film-coated tablets obtained in respective examples was measured according to the testing method for uniformity of content described in 14th revision Japanese pharmacopoeia. As a result, all were fitted for the acceptance value of testing method for content uniformity, hence the content of KRP-101 was uniform. The results are shown in Table 1.

### [Table 1]

**(Table 1) Content uniformity of KRP-101 film-coated tablets**

| | Example 1 | Example 2 | Example 3 |
|---|---|---|---|
| Average value (%) | 95.3 | 97.6 | 93.6 |
| Range (%) | 94.3 - 97.0 | 94.7 - 101.0 | 92.5 - 94.7 |
| Acceptance value (%) | 6.6 | 7.0 | 8.0 |

| | | | |
|---|---|---|---|
| Acceptance value: Not more than 15.0%. | | | |

### <Experimental example 2>

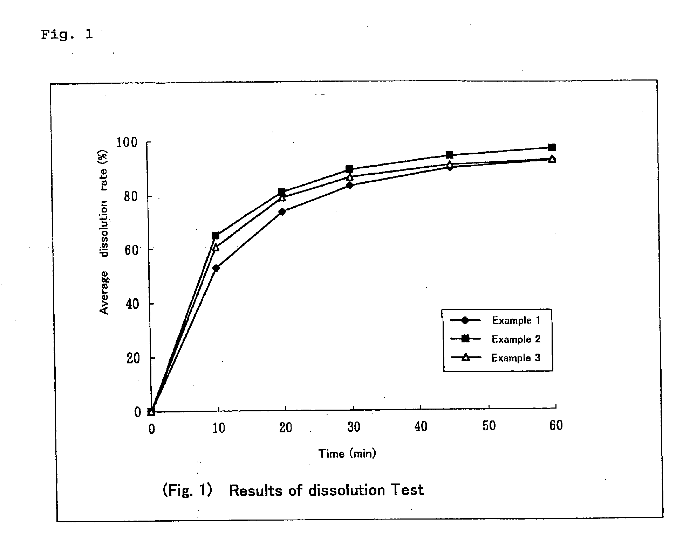

As a result of testing the film-coated tablets obtained in respective examples according to the dissolution testing method, 2nd method (rotation speed: 50 rpm, dissolution media: diluted phosphate buffer with pH 6.8, 900 mL) described in 14th revision Japanese pharmacopoeia, all showed 80% or more average dissolution rate (n = 6) for 30 minutes, hence the dissolution of KRP-101 from tablet was rapid. The results are shown in Fig. 1.

### <Experimental example 3>

As a result of performing the measurement of hardness, friability and disintegration time (disintegration media: water) of the prime tablets obtained in respective examples, all showed acceptable = values in pharmaceutical properties. The results are shown in Table 2.

### [Table 2]

**(Table 2) Pharmaceutical properties of KRP-101 plain tablets**

| | Example 1 | Example 2 | Example 3 |
|---|---|---|---|
| Hardness (kg) | 4.32 | 5.44 | 4.71 |
| Friability (%) | 0.1 | 0.2 | 0.1 |
| Disintegration time (sec) | 33 | 19 | 26 |

### <Experimental example 4>

To a male Beagle dog, one tablet of the film-coated tablets obtained in Example 1 was administered orally or 0.5mg/kg of KRP-101 were administered intravenously,andthe concentration of KRP-101 in plasma was measured until 24 hours after administration. As a result, the bioavailability of the film-coated tablets obtained in Example 1 showed a value as high as 85%, hence it was inferred that KRP-101 released from the film-coated tablet was absorbed well from gastrointestinal tract. The results are shown in Table 3.

### [Table 3]

**(Table 3) Pharmacokinetic parameters of KRP-101 on administering orally (one tablet of the film-coated tablets obtained in Example 1: 1mg/body) and intravenously (0.5mg/kg) to a male Beagle dog**

| | Oral administration (Example 1) | Intravenous administration |
|---|---|---|
| Cmax (ng/mL) | 224 | ― |
| Tmax (hour) | 2.7 | ― |
| T1/2 (hour) | 3.2 | 2.2 |
| AUC (ng · hour/mL ) ) | 1191 | 1354 |
| BA (%) | 85 | ― |

| | | |
|---|---|---|
| Cmax: Maximum concentration in plasma, | | |
| Tmax: Time to reach maximum concentration in plasma, | | |
| T1/2: Elimination half-life, | | |
| AUC: Total area under concentration-time curve, | | |
| BA: Bioavailability. | | |

### Industrial applicability

In accordance with the invention, film-coated tablets uniformly containing a small amount of KRP-101 can be obtained, making it possible to orally administer a microdose of KRP-101 quantitatively on clinical study.

### Brief description of drawing

[Fig. 1] Results of dissolution test of respective film-coated tablets performed in Experimental example 2.

## Claims

1. An oral solid dosage form having (S)-2-[3-[N-[4-(4-fluorophenoxy)benzyl]carbamoyl]-4-methoxybenz yl]butanoic acid (hereinafter abbreviated as KRP-101) as an effective ingredient and comprising KRP-101 and additives.

2. The oral solid dosage form of Claim 1, wherein the additives comprise excipient, disintegrator and lubricant, or these and coating agent.

3. The oral solid dosage form of Claim 1 or 2, wherein the excipient comprises lactose and/or microcrystalline cellulose, the disintegrator comprises low substituted hydroxypropylcellulose, the lubricant comprises magnesium stearate, and the coating agent comprises hydroxypropylmethylcellulose and/or carnauba wax.

4. The oral solid dosage form of any of Claim 1 through 3, wherein, to a mixed powder obtained by repeating a plurality of steps of mixing and dilution of KRP-101 with excipient, the excipient, disintegrator and lubricant are added and the mixed powder with less than 1% of KRP-101 is granulated.
